# EUROPEAN PATENT APPLICATION

(11) **EP 4 730 358 A1**
(43) Date of publication of application: **22.04.2026**
(21) Application number: 24207021.7
(22) Date of filing: 16.10.2024
(51) Int. Cl.: G16H 50/50, G16H 50/20, G16H 30/40

(54) **PERFORMING A HAEMODYNAMIC SIMULATION**

(71) Applicant: Koninklijke Philips N.V., 5656 AG Eindhoven (NL)
(72) Inventor: HAASE, Hans Christian, 5656 AG Eindhoven (NL); GRASS, Michael, 5656 AG Eindhoven (NL)
(74) Representative: Philips Intellectual Property & Standards

(57) **Abstract**

A computer-implemented method of performing a haemodynamic simulation, is provided. The method includes: receiving, by a first processing system (110), spectral CT data (120) representing an anatomical region (130). The method also includes performing (S120), using the first processing system (110), a first haemodynamic simulation using at least a portion of the spectral CT data (120) to provide a first haemodynamic simulation result (140). The method also includes obtaining (S130) a reduced spectral CT dataset (120') from the spectral CT data (120) based on a relevance of one or more subsets (120_{1..i}) of the spectral CT data to the first haemodynamic simulation result (140). The method also includes transmitting (S140) the reduced spectral CT dataset (120') to a second processing system (150) and/or to a computer-readable storage medium (160).

## Description

### FIELD OF THE INVENTION

The present disclosure relates to performing a haemodynamic simulation. A computer-implemented method, a computer program product, a system, and a processing arrangement, are disclosed.

### BACKGROUND OF THE INVENTION

Haemodynamic simulations are often performed during clinical investigations relating to the vasculature. For instance, haemodynamic simulations are often performed during clinical investigations for coronary artery disease "CAD". The result of performing a haemodynamic simulation is typically a value of a haemodynamic parameter, or a clinical diagnosis. For instance, a haemodynamic simulation for a coronary artery may result in the value of a haemodynamic parameter for the artery, or an indication of the presence, or the severity, of CAD in the artery. Examples of haemodynamic parameters that may be evaluated in a haemodynamic simulation include: a blood pressure, a blood flow, a blood flow resistance, the Fractional Flow Reserve "FFR", the instantaneous wave-free ratio "iFR", the Coronary Flow Reserve "CFR", the Thrombolysis in Myocardial Infarction "TIMI" flow grade, the Index of Microvascular Resistance "IMR", and the Hyperemic Microvascular Resistance index "HMR", and so forth.

Historically, haemodynamic simulations have been performed using conventional computed tomography "CT" images. Increasingly, spectral CT images are being used to perform haemodynamic simulations. Spectral CT images have many advantages over conventional CT images. For instance, spectral CT images offer improved material differentiation over conventional CT images. This in-turn enables diagnoses to be performed that would be harder, if not impossible, to perform from conventional CT images. However, these advantages incur a cost. The spectral CT datasets of spectral CT images are typically much larger than the datasets of conventional CT images. This is because spectral CT datasets represent X-ray attenuation within multiple energy intervals, whereas conventional CT datasets, by contrast, represent X-ray attenuation within only a single energy interval.

The size of spectral CT datasets presents challenges, particularly when they are transmitted. Spectral CT datasets may need to be transmitted for various reasons, such as to perform further analysis on the dataset, and to store the dataset. For instance, a spectral CT dataset may need to be transmitted from a processing system that is local to its acquisition to a remote, e.g. a cloud-based, processing system that has greater processing power in order to perform further analysis on the dataset. A spectral CT dataset may also need to be transmitted from a processing system to a data storage facility, such as a Picture Archiving and Communication System "PACS". A spectral CT dataset may also need to be transmitted from a data storage facility, such as a PACS system. For instance, a spectral CT dataset may need to be transmitted from a data storage facility for further analysis, or for storage elsewhere, e.g. in the form of a radiology report. The transmission of spectral CT datasets in situations such as these can be challenging due to bandwidth limitations in the transmission medium, and this can lead to delays in their processing, and in their storage.

Consequently, there is a need to reduce the size of spectral CT datasets.

### SUMMARY OF THE INVENTION

According to one aspect of the present disclosure, a computer-implemented method of performing a haemodynamic simulation, is provided. The method includes:
- receiving, by a first processing system, spectral CT data representing an anatomical region;
- performing, using the first processing system, a first haemodynamic simulation using at least a portion of the spectral CT data to provide a first haemodynamic simulation result;
- obtaining a reduced spectral CT dataset from the spectral CT data based on a relevance of one or more subsets of the spectral CT data to the first haemodynamic simulation result; and
- transmitting the reduced spectral CT dataset to a second processing system and/or to a computer-readable storage medium.

The above method is based on the insight that whilst a spectral CT dataset provides more information on an anatomical region than a conventional CT dataset, not all of the data in a spectral CT dataset provides useful information. In the above method, a reduced spectral CT dataset is obtained and transmitted to a second processing system and/or a computer-readable storage medium. This alleviates bandwidth limitations and/or delays incurred in transmitting the data. Since the reduced spectral CT dataset is obtained from the spectral CT data based on a relevance of one or more subsets of the spectral CT data to the first haemodynamic simulation result, it is ensured that the reduced dataset that is transmitted, is relevant to the haemodynamic simulation result.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of performing a haemodynamic simulation, in accordance with some aspects of the present disclosure.
Fig. 2 is a schematic diagram illustrating an example of a processing arrangement 200 for performing a haemodynamic simulation, in accordance with some aspects of the present disclosure.
Fig. 3 is a schematic diagram illustrating an example of various elements of a method of performing a haemodynamic simulation, in accordance with some aspects of the present disclosure.

### DETAILED DESCRIPTION OF THE EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer-implemented method, may be implemented in a computer program product, and in a system, and in a processing arrangement, in a corresponding manner.

In the following description, reference is made to a computer-implemented method of performing a haemodynamic simulation. Reference is made to examples in which a haemodynamic simulation is performed for a cardiac artery, i.e. a portion of the cardiac vasculature. However, it is to be appreciated that the method may be used to perform haemodynamic simulations for portions of the vasculature that are in other anatomical regions than the heart. For instance, the method may be used to perform a haemodynamic simulation for portions of the vasculature that are in the brain, the lung, the kidney, the arm, the leg, and so forth. Moreover, it is to be appreciated that the method may be used to perform haemodynamic simulations for blood vessels other than an artery. For instance, the method may be used to perform haemodynamic simulations for a vein, or an arteriole, or a capillary, or a venule, or a network thereof.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, or processing arrangement, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed in the computer-implemented methods disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations performed in the computer-implemented methods disclosed herein.

As mentioned above, there is a need to reduce the size of spectral CT datasets.

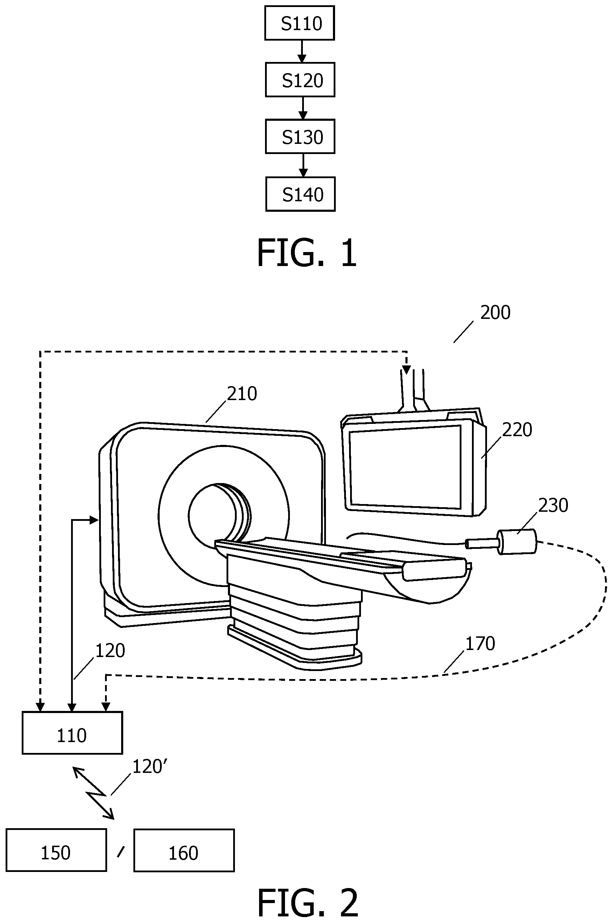

Fig. 1 is a flowchart illustrating an example of a method of performing a haemodynamic simulation, in accordance with some aspects of the present disclosure. Fig. 2 is a schematic diagram illustrating an example of a processing arrangement 200 for performing a haemodynamic simulation, in accordance with some aspects of the present disclosure. It is noted that operations that are described in relation to the method illustrated in Fig. 1, may also be performed by the processing arrangement 200 illustrated in Fig. 2. Likewise, operations that are described as being performed by the processing arrangement 200 illustrated in Fig. 2, may also be performed in the method described with reference to Fig. 1. With reference to Fig. 1, and Fig. 2, the computer-implemented method of performing a haemodynamic simulation, includes:
- receiving S110, by a first processing system 110, spectral CT data 120 representing an anatomical region 130;
- performing S 120, using the first processing system 110, a first haemodynamic simulation using at least a portion of the spectral CT data 120 to provide a first haemodynamic simulation result 140;
- obtaining S130 a reduced spectral CT dataset 120' from the spectral CT data 120 based on a relevance of one or more subsets 120_{1..i} of the spectral CT data to the first haemodynamic simulation result 140; and
- transmitting S140 the reduced spectral CT dataset 120' to a second processing system 150 and/or to a computer-readable storage medium 160.

The above method is based on the insight that whilst a spectral CT dataset provides more information on an anatomical region than a conventional CT dataset, not all of the data in a spectral CT dataset provides useful information. In the above method, a reduced spectral CT dataset is obtained and transmitted to a second processing system and/or a computer-readable storage medium. This alleviates bandwidth limitations and/or delays incurred in transmitting the data. Since the reduced spectral CT dataset is obtained from the spectral CT data based on a relevance of one or more subsets of the spectral CT data to the first haemodynamic simulation result, it is ensured that the reduced dataset that is transmitted, is relevant to the haemodynamic simulation result.

The operations that are performed in the method illustrated in Fig. 1, are described in more detail below.

Referring initially to the operation S 110; in this operation, spectral CT data 120 representing an anatomical region 130 is received by a first processing system 110. The spectral CT data 120 that is received in the operation S110 defines the X-ray attenuation of the anatomical region within each of a plurality of different energy intervals. In general there may be two or more energy intervals. By processing the data from the plurality of different energy intervals (e.g. using various material decomposition algorithms), a distinction can be made between materials that have similar X-ray attenuation values when measured within a single energy interval, and which would be indistinguishable from attenuation data generated by a conventional CT imaging system.

The spectral CT data 120 that is received in the operation S 110 may be generated by various configurations of spectral X-ray imaging systems, some of which are described below. In general, these include spectral CT imaging systems that acquire spectral X-ray attenuation data from each of a plurality of rotational angles with respect to an anatomical region whilst rotating an X-ray source-detector arrangement around the anatomical region, and also spectral X-ray projection imaging systems that are configured to acquire spectral X-ray attenuation data from each of a plurality of rotational angles with respect to an anatomical region. A spectral X-ray projection imaging systems is typically configured to acquire spectral X-ray attenuation data with its source-detector arrangement in a fixed position with respect to an anatomical region. However, the X-ray source-detector arrangement of a spectral X-ray projection imaging systems may be configured to acquire spectral X-ray attenuation data from each of a plurality of rotational angles with respect to an anatomical region by stepping, or by rotating, its X-ray source-detector arrangement around the anatomical region.

In general, a spectral CT imaging system and a spectral X-ray projection imaging system are distinguished from their conventional CT imaging system and conventional X-ray projection imaging system counterparts by their ability to generate X-ray attenuation data within each of a plurality of different energy intervals. The set of spectral X-ray attenuation data that is acquired from a plurality of rotational angles by a spectral X-ray projection imaging system may be referred-to as spectral CT data because this dataset may be reconstructed to provide a volumetric image using similar techniques to those that are used to reconstruct the spectral CT data that is generated by a spectral CT imaging system.

In general, the X-ray source in the X-ray source-detector arrangement in the above-mentioned configurations of spectral X-ray imaging systems may be provided by multiple monochromatic sources, or by one or more polychromatic sources. In general, the X-ray detector in the X-ray source-detector arrangement in these configurations may include: a common detector for detecting X-ray radiation across multiple different X-ray energy intervals, or multiple detectors wherein each detector detects X-ray radiation within a different X-ray energy interval, or a multi-layer detector in which X-ray radiation within each of a plurality of different X-ray energy intervals is detected by corresponding layers, or a photon counting detector that bins detected X-ray photons into one of a plurality of different energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various techniques may be used to generate X-ray attenuation data within each of a plurality of different X-ray energy intervals in the above-mentioned configurations. These include performing a temporal modulation of the X-ray spectrum; for example by temporally switching the X-ray anode potential of a single X-ray source, a technique known as "rapid kVp switching"; and temporally switching, or filtering, the emission of X-rays from multiple X-ray sources. Techniques that perform a temporal modulation of the X-ray spectrum may employ a common X-ray detector to detect X-rays across a plurality of different energy intervals, attenuation data for each of the energy intervals being generated in a time-sequential manner. Instead of performing a temporal modulation of the X-ray spectrum, a discrimination between different X-ray energy intervals may be provided at the X-ray detector by using a multi-layer detector, or a photon counting detector. Such detectors can detect X-rays from each of a plurality of different X-ray energy intervals near-simultaneously, and thus there is no need to perform temporal modulation of the X-ray spectrum. Thus, a multi-layer X-ray detector, or a photon counting X-ray detector, may be used in combination with a polychromatic X-ray source to generate spectral attenuation data within each of a plurality of different energy intervals.

Other configurations of the aforementioned X-ray sources and X-ray detectors may alternatively be used in a spectral CT imaging system, or in a spectral X-ray projection imaging system, to generate X-ray attenuation data within each of a plurality of different X-ray energy intervals. For example, in a yet further configuration, the need to sequentially switch different X-ray sources emitting X-rays in different energy intervals may be obviated by acquiring spectral X-ray attenuation data using two rotationally-offset X-ray source-detector arrangements. In this configuration, each X-ray source-detector arrangement acquires spectral X-ray attenuation data for a different X-ray energy interval, and so the spectral X-ray attenuation data for two different X-ray energy intervals may be acquired simultaneously.

In yet another configuration, spectral CT data 120 may be acquired from a conventional CT imaging system, or a conventional X-ray projection imaging system, by intercepting its X-ray beam with one or more spectral filters. For instance, a single filter may be mechanically moved into, and out of, the X-ray beam generated by a polychromatic X-ray source in order to temporally modulate the spectrum of X-rays that are detected by the corresponding X-ray detector. The filter transmits only a portion of the spectrum of X-rays emitted by the polychromatic X-ray source, and thus provides X-ray attenuation data for a first energy interval when the filter intercepts the beam. When the filter is removed from the beam, X-ray attenuation data is provided for a second energy interval, e.g. for the complete spectrum emitted by the polychromatic X-ray source. In so doing, X-ray attenuation data for each of the energy intervals is generated in a time-sequential manner. In this configuration, multiple filters, each with a different X-ray transmission spectrum, may be sequentially switched into, and out of, the X-ray beam in order to increase the number of energy intervals.

The spectral CT data 120 that is received in the operation S110 may represent various anatomical regions. For instance, the spectral CT data may represent a portion of the heart, or a portion of the brain, or a portion of the lung, or a portion of the kidney, or a portion of the arm, or a portion of the leg, and so forth. In general, the anatomical region includes a portion of the vasculature. In this regard, the portion of the vasculature may include one or more blood vessels. For instance, it may include one or more arteries, one or more veins, one or more arterioles, one or more capillaries, one or more venules, and so forth. The portion of the vasculature may include a network of such blood vessels, for example.

The spectral CT data 120 that is received in the operation S110 may be generated subsequent to the injection of a contrast agent into the vasculature. The contrast agent may include a substance such as Iodine, or a Lanthanide such as Gadolinium, or another substance that provides visibility of a blood flow into which the contrast agent is injected. The contrast agent may be injected manually or automatically, e.g. via an injector.

The spectral CT data 120 that is received in the operation S110 may correspond to a single volumetric image of the anatomical region, or alternatively it may correspond to multiple volumetric images of the anatomical region, e.g. a temporal sequence of volumetric images of the anatomical region. The temporal sequence may represent a flow of an injected contrast agent bolus, for example. The images may be fluoroscopic images.

The spectral CT data 120 that is received in the operation S110 may be raw data, i.e. data that has not been reconstructed into volumetric image, or alternatively it may be image data, i.e. data that represents a reconstructed volumetric image.

The spectral CT data 120 that is received in the operation S110 is received by the first processing system 110. In general, the first processing system 110 includes one or more processors. The first processing system 110 may be provided by a computer such as a tablet, or a laptop, or a desktop computer, or a mainframe computer. The first processing system 110 may alternatively be provided in the form of a mobile communication device, such as a mobile telephone. In some examples, the first processing system 110 the first processing system 110 is local to the spectral CT imaging system, or the spectral X-ray projection imaging system, that generates the spectral CT data 120. For instance, in the example illustrated in Fig. 2, the first processing system 110 is local to the spectral CT imaging system 210, i.e. located within the same medical facility.

The spectral CT data 120 that is received in the operation S110 may be received from various sources. For example, the spectral CT data 120 may be received from a spectral CT imaging system, or from a spectral X-ray projection imaging system, that generates the spectral CT data 120. In these examples, the spectral CT data 120 may be received from a computer readable storage medium that is in communication with the spectral CT imaging system, or the spectral X-ray projection imaging system, respectively. More generally, the spectral CT data 120 that is received in the operation S110 may be received from a computer readable storage medium. In general, the computer readable storage medium may be in any location. For instance it may be local to the spectral CT imaging system, or the spectral X-ray projection imaging system, or it may be remote, e.g. in the Cloud. In one example, the spectral CT data 120 that is received in the operation S110 is received from a computer readable storage medium that serves as a PACS system. A spectral CT dataset may need to be transmitted from the PACS system for further analysis, or for storage elsewhere, e.g. in the form of a radiology report.

In general, the spectral CT data 120 that is received in the operation S110 may be received via any form of data communication. For instance, the spectral CT data may be received via wired, or optical, or wireless communication. By way of some examples, when wired or optical communication is used, the communication may take place via signals transmitted on an electrical or optical cable, and when wireless communication is used, the communication may take place via RF or optical signals.

Referring now to the operation S120 illustrated in Fig. 1; in this operation, the first processing system 110 performs a first haemodynamic simulation using at least a portion of the spectral CT data 120. This provides a first haemodynamic simulation result 140.

The first haemodynamic simulation may be performed in various ways in the operation S120. For instance, in one example, the spectral CT data is segmented in order to identify anatomical structure(s), and the value of a haemodynamic parameter is calculated for the anatomical structure(s). In this example, the operation of performing S120 a first haemodynamic simulation includes:
- applying one or more segmentation operations to the at least a portion of the spectral CT data 120 to identify one or more anatomical structures 120_{1..i} in the anatomical region 130; and
- calculating a value of a haemodynamic parameter for at least one of the one or more anatomical structures 120_{1..i} to provide the first haemodynamic simulation result 140.

The segmentation operation(s) that are performed in this example may include segmentation(s) such as a model-based segmentation, a watershed-based segmentation, a neural network-based segmentation, or other segmentation operations such as region growing, level sets, graphcuts, and so forth. The segmentation operation(s) may be used to identify anatomical structures such as one or more blood vessels for which the haemodynamic parameter is to be evaluated. For instance, the segmentation operation(s) may be used to identify a coronary artery for which an FFR value is to be evaluated.

The segmentation operation(s) that are performed in this example may be applied to only a portion of the spectral CT data. In this regard, the spatial extent of the anatomical region for which the spectral CT data is segmented, or the spectral extent of the CT data that is processed, may represent a subset of the complete set of spectral CT data. For instance, the segmentation operation(s) may be applied only within a portion of the anatomical region 130 that corresponds to the myocardium, or to only a subset of the energy intervals that are represented by the spectral CT data 120. For instance, the segmentation operation may be applied only to a portion of the spectral CT data that represents a so-called Iodine image (i.e. the contrast agent distribution in the anatomical region), or only to a monoenergetic 70 keV equivalent "MonoE70" image. Alternatively, the segmentation operation(s) that are performed in this example may be applied to the complete set of spectral CT data. For instance, the segmentation operation(s) may be applied to all anatomical regions, or to all energy intervals that are represented by the spectral CT data 120, or to a weighted sum of all energy intervals that are represented by the spectral CT data 120. Applying the segmentation operation(s) to a portion of the spectral CT data simplifies the evaluation of the haemodynamic parameter.

Various haemodynamic parameters may be evaluated in accordance with this example. For instance, the value of a blood pressure, blood flow, or blood flow resistance may be calculated. Some further examples of haemodynamic parameters that may be evaluated include the FFR, the iFR, the CFR, a TIMI flow grade, the IMR, and the HMR. The value of the haemodynamic parameter may be evaluated using various models, such as a haemodynamic model, or a lumped parameter model.

In one example, a haemodynamic model is used to calculate the value of the haemodynamic parameter. A haemodynamic model may be generated using the geometry of the anatomical structure(s) that are identified in the above-mentioned segmentation operation(s). For instance, the haemodynamic model may represent the geometry of the vasculature in the anatomical region via the 3D centerline of the vessel(s) with elliptic cross-sections along the length of the 3D centerline(s). The hemodynamic model may be based on Navier-Stokes partial differential equations. The model may then be solved using CFD techniques to simulate the blood flow in a vessel and to evaluate haemodynamic parameter(s) such as those mentioned above.

In another example, a lumped parameter model is used to used to calculate the value of the haemodynamic parameter. A lumped parameter model represents blood flow in a vascular region in a similar manner to an electrical circuit; a volumetric flow rate of the blood being represented as an electrical current, a pressure of the blood being represented as a voltage, and a blood volume being represented by an electrical charge. In a lumped parameter model, resistance to blood flow is represented by a linear or nonlinear electrical resistor, vessel wall compliance is represented by a capacitance, and blood inertia is represented by an inductance. A machine learning-based approach for setting the values of linear and nonlinear resistors in a static lumped parameter model based on angiographic geometric measurements of the vessels in the vascular region that may be used to calculate the value of the above-mentioned haemodynamic parameter for a vessel is disclosed in a document by Nickisch, H., et al., "Learning Patient-Specific Lumped Models for Interactive Coronary Blood Flow Simulations", MICCAI 2015, Part II, LNCS 9350, pp. 433 - 441, 2015, and also in the document WO 2016/001017. As compared to the use of CFD techniques, the use of a lumped parameter model to evaluate haemodynamic parameters offers a reduction in both computational burden and model complexity.

The result of calculating a value of a haemodynamic parameter for the anatomical structure(s) in this example is to provide a first haemodynamic simulation result 140. The first haemodynamic simulation result may be simply the value of the haemodynamic parameter. Alternatively or additionally, the first haemodynamic simulation result may be a clinical diagnosis of the anatomical region 130. The clinical diagnosis can be made based on the first haemodynamic simulation, e.g. based on the value of the haemodynamic parameter. For instance, an FFR may be evaluated for a vessel, and the FFR may be used to conclude that the vessel has a stenosis, or that CAD is present, or that the severity of CAD is at a specified level.

In another example, the first haemodynamic simulation that is performed in the operation S 120 uses a machine learning model. In this example, the operation of performing S 120 a first haemodynamic simulation includes:
- inputting the at least a portion of the spectral CT data 120 into a machine learning model; and
- generating the first haemodynamic simulation result 140 in response to the inputting, using the machine learning model; and
- wherein the machine learning model is trained to generate the first haemodynamic simulation result 140 based on the inputted spectral CT data 120.

As in the previous example, the first haemodynamic simulation result that is generated in this example may be the value of a haemodynamic parameter. In this case, the training data that is used to train the machine learning model may include instances of spectral CT data, e.g. multiple spectral CT images, and corresponding ground truth data in the form of values of haemodynamic parameters for various vessels. Alternatively or additionally, in this example, the first haemodynamic simulation result may be a clinical diagnosis of the anatomical region. The clinical diagnosis can be made based on the first haemodynamic simulation, e.g. based on the value of the haemodynamic parameter. For instance, an FFR may be evaluated for a vessel, and the FFR may be used to conclude that the vessel has a stenosis, or that CAD is present, or that the severity of CAD is at a specified level. A clinical diagnosis can alternatively be made independently of any value of a haemodynamic parameter. For instance, the machine learning model may simply determine that a vessel in the anatomical region has a stenosis, or that CAD is present, or that the severity of CAD is at a specified level. In this case, the training data that is used to train the machine learning model may include instances of spectral CT data, e.g. multiple spectral CT images, and corresponding ground truth data in the form of a corresponding diagnosis, such as an indication of the location of a stenosis in a vessel in the anatomical region.

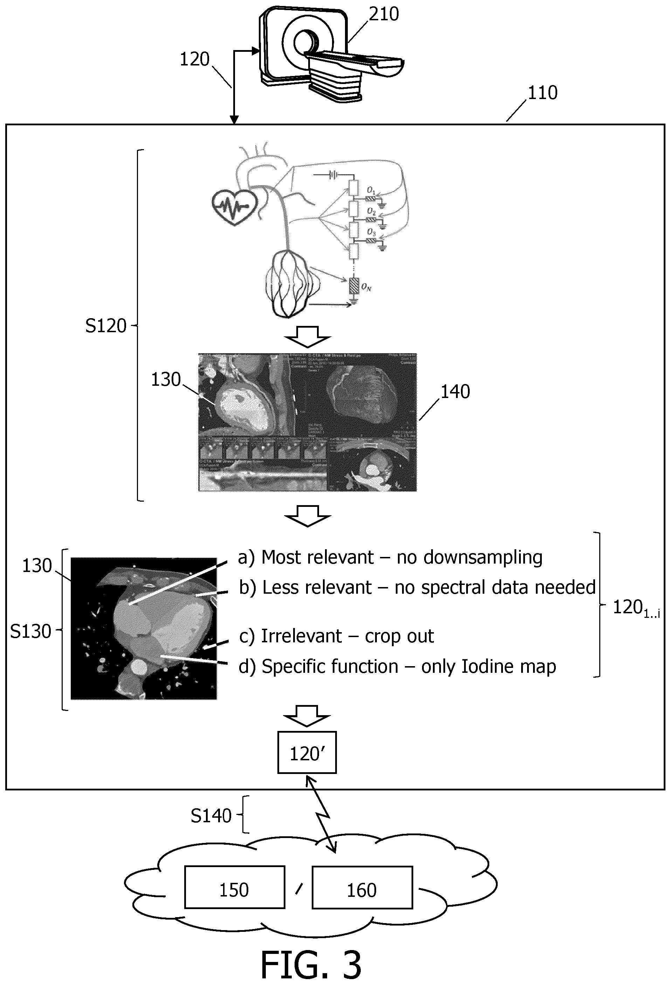

An example of the operation S 120 is illustrated in Fig. 3, which is a schematic diagram illustrating an example of various elements of a method of performing a haemodynamic simulation, in accordance with some aspects of the present disclosure. In the example illustrated in Fig. 3, the spectral CT data 120 is generated by the spectral CT imaging system 120. The spectral CT data 120 is then received by the one or more processors 110. In the operation S120, a lumped parameter model representing a network of cardiac vessels in the spectral CT data is used to perform a first haemodynamic simulation, and thereby provide a first haemodynamic simulation result 140. In this example, the first haemodynamic simulation result 140 includes a value of a haemodynamic parameter (e.g. an FFR value) for a vessel in the network of cardiac vessels.

Referring now to the operation S 130 illustrated in Fig. 1; in this operation, a reduced spectral CT dataset 120' is obtained from the spectral CT data 120 based on a relevance of one or more subsets 120_{1..i} of the spectral CT data to the first haemodynamic simulation result 140. In general, the reduced spectral CT dataset 120' that is obtained in the operation S130 has a smaller size than the spectral CT data 120.

The reduced spectral CT dataset 120' is obtained from the spectral CT data 120 based on a relevance of one or more subsets 120_{1..i} of the spectral CT data to the first haemodynamic simulation result 140. In one example, the subset(s) of the spectral CT data correspond to one or more anatomical structures in the anatomical region 130. In another example, the subset(s) correspond to one or more energy intervals of the spectral CT data. Alternatively, the subset(s) may correspond to a combination of anatomical structure(s) and energy interval(s). The operation S130 may be performed by the first processing system 110.

In one example, the operation of obtaining S 130 a reduced spectral CT dataset 120' from the spectral CT data 120 includes determining a relevance of the one or more subsets 120_{1..i} of the spectral CT data 120 to the first haemodynamic simulation result 140; and selecting, based on the relevance of the one or more subsets 120_{1..i}, at least one of the one or more subsets to provide the reduced spectral CT dataset 120'.

For instance, in this example, if the Lateral Anterior Descending "LAD" artery, i.e. an anatomical structure, is deemed to be the most relevant anatomical structure to an FFR value, i.e. a first haemodynamic simulation result, then the reduced spectral CT dataset may be obtained by selecting only the spectral CT data corresponding to the LAD artery. If the feeding territory of the LAD artery, i.e. another anatomical structure, is also deemed to be highly relevant to the FFR value, then the spectral CT data corresponding to the feeding territory may also be included in the reduced spectral CT dataset. By way of another example, if the first haemodynamic simulation result is a determination that microvascular disease is present, i.e. a clinical diagnosis, then the spectral CT data corresponding to the myocardium may be included in the reduced spectral CT dataset.

Instead of, or in addition to, selecting subset(s) of the spectral CT data 120 that correspond to anatomical structure(s) to obtain the reduced spectral CT dataset, the reduced spectral CT dataset may be obtained by selecting subset(s) of the spectral CT data 120 that correspond to one or more energy intervals. For instance, if an Iodine image, or a "MonoE70" image, is deemed to represent the most relevant range of energy intervals to the FFR value, then the reduced spectral CT dataset may be obtained by selecting only the spectral CT data corresponding to the Iodine image, or the "MonoE70" image.

In another example, the operation of obtaining S130 a reduced spectral CT dataset 120' from the spectral CT data 120 includes determining a relevance of the one or more subsets 120_{1..i} of the spectral CT data 120 to the first haemodynamic simulation result 140; and compressing at least a portion of the spectral CT data 120 based on the relevance of the one or more subsets 120_{1..i} to provide the reduced spectral CT dataset 120'.

For instance, in this example, if the Lateral Anterior Descending "LAD" artery, i.e. an anatomical structure, is deemed to be the most relevant anatomical structure to an FFR value, i.e. a first haemodynamic simulation result, then the reduced spectral CT dataset may be obtained by compressing the spectral CT data for anatomical regions other than the LAD artery and by not compressing, or compressing by a lesser degree, the spectral CT data for the LAD artery. Likewise, if the feeding territory of the LAD artery, i.e. another anatomical structure, is also deemed to be highly relevant to the FFR value, then the spectral CT data corresponding to the feeding territory may not be compressed, or may be compressed to a lesser degree, than the spectral CT data for anatomical regions other than the feeding territory and the LAD artery.

Instead of, or in addition to, compressing the spectral CT data based on the relevance of anatomical structures to the first haemodynamic simulation result in order to obtain the reduced spectral CT dataset, the reduced spectral CT dataset may be obtained by compressing the spectral CT data based on the relevance of the energy intervals to the first haemodynamic simulation result. For instance, if an Iodine image is deemed to represent the most relevant range of energy intervals to the FFR value, then the reduced spectral CT dataset may be obtained by compressing the spectral CT data for energy intervals other than the Iodine image, and not compressing, or compressing by a lesser degree, the spectral CT data for the Iodine image.

The relevance of one or more subsets 120_{1..i} of the spectral CT data to the first haemodynamic simulation result 140 may be determined in various ways in the operation S130. For instance, in one example, the relevance is determined from a look-up table. The look-up table may provide for each of multiple instances of the first haemodynamic simulation result, the corresponding relevant subset(s) of the spectral CT data. For example, if the first haemodynamic simulation result is an FFR value, then the look-up table may provide a list of the relevant portions of the cardiac vasculature and/or relevant energy interval(s) of the spectral CT data, for different FFR values. Thus, for a relatively lower FFR value, the list of relevant portions of the cardiac vasculature may be only the vessel in which the FFR value is minimum and the relevant energy interval may be only that corresponding to an Iodine image, whereas for a relatively higher FFR value, the list of relevant portions of the cardiac vasculature may also include a portion of the feeding territory that feeds the vessel, and the relevant energy intervals may be all energy intervals. Similarly, if the first haemodynamic simulation result is a clinical diagnosis, such as a conclusion that a vessel has a stenosis, then the look-up table may provide a list of the relevant portions of the cardiac vasculature and/or relevant energy intervals of the spectral CT data, for different degrees of stenosis.

Instead of determining the relevance of one or more subsets 120_{1..i} of the spectral CT data to the first haemodynamic simulation result in the operation S 130 using a look-up table, a heatmap, or an overlay mask, may be used. For instance, if the spectral CT data represents a reconstructed image, then a heatmap or an overlay mask may be used to define the anatomical region(s) of the reconstructed image that are relevant to various clinical diagnoses, i.e. a first haemodynamic simulation result. The heatmap, or the overlay mask, may be stored in the form of a library of templates for different clinical diagnoses. The heatmap indicates, in an analogue, or in a binary manner, the relative importance of one or more anatomical structures in the anatomical region 130 to the first haemodynamic simulation result 140 and/or a relative importance of one or more energy intervals of the spectral CT data 120 to the first haemodynamic simulation result 140.The overlay mask, by contrast, indicates the relative importance in a binary manner. Thus, if for example the clinical diagnosis is the conclusion that a stenosis is present in a cardiac vessel in the anatomical region, then the heatmap, or the overlay mask, that is relevant to a stenosis in that vessel may be retrieved, and applied to the reconstructed image in order to define the volumetric extent of the spectral CT data that is relevant to the clinical diagnosis, and thereby obtain the reduced spectral CT dataset.

Thus, in one example, a heatmap is used to obtain the reduced spectral CT dataset 120' in the operation S130. In this example, the heatmap indicates a relative importance of one or more anatomical structures in the anatomical region 130 to the first haemodynamic simulation result 140 and/or a relative importance of one or more energy intervals of the spectral CT data 120 to the first haemodynamic simulation result 140. The operation of obtaining S130 a reduced spectral CT dataset 120', comprises using the heatmap to determine the relevance of the one or more subsets 120_{1..i} of the spectral CT data 120 to the first haemodynamic simulation result 140.

Instead of retrieving the heatmap from a library, the heatmap may instead be generated by the above-mentioned machine-learning model. Thus, in one example, the machine learning model is further trained to generate the heatmap; and the heatmap is generated in response to inputting the at least a portion of the spectral CT data 120 into the machine learning model.

For instance, if the portion of the spectral CT data 120 that is inputted into the machine learning model is a reconstructed image representing the anatomical region, then the machine learning model may generate the heatmap in the form of an overlay onto the volumetric image. Various techniques that are known from the field of machine learning may be used to generate the heatmap in this example. Generating the heatmap from the machine learning model enables the determination of the relevant subset(s) of the spectral CT data in a straightforward and reliable manner.

An example of the operation S 130 is illustrated in Fig. 3. This example continues from the above description of the operation S 120 which provided the first haemodynamic simulation result 140 (e.g. a value of a haemodynamic parameter). In the operation S 130, the relevance of various subsets 120_{1..i} of the spectral CT data to the first haemodynamic simulation result 140, is then used to obtain the reduced spectral CT data set 120'. The relevance of the anatomical regions a) - d) is labelled in Fig. 3. The relevance of these anatomical regions has been determined using a look-up table. Thus, the anatomical region a) has been deemed to be the most relevant. The anatomical region b) has been deemed to be less relevant. The anatomical region c) has been deemed to be irrelevant. The anatomical region d) has been deemed to be partially relevant. The relevance of the anatomical regions a) - d) is then used to obtain the reduced spectral CT dataset 120'. Thus, due to its high level of relevance, the spectral CT data corresponding to the anatomical region a) is included in the reduced spectral CT dataset 120' without any compression at all. Due to its lesser relevance, the amount of data that represents each voxel within the anatomical region b) is reduced by applying a weighted average to the X-ray attenuation values of all X-ray energy intervals for the voxel and thereby obtaining an average X-ray attenuation value for the voxel. Due to its irrelevance, the spectral CT data corresponding to the anatomical region c) is omitted from the reduced spectral CT dataset 120'. Due to its partial relevance, only the spectral CT data corresponding to the Iodine map of the anatomical region d) is included in the reduced spectral CT dataset 120'.

Referring now to the operation S 140 illustrated in Fig. 1; in this operation, the reduced spectral CT dataset 120' is transmitted to a second processing system 150 and/or to a computer-readable storage medium 160.

In general, the second processing system 150 includes one or more processors. In general, the computer-readable storage medium 160 may include any type of computer-readable storage medium. For instance, as mentioned above, the computer-readable storage medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD. The computer-readable storage medium 160 may be a Picture Archiving and Communication System "PACS", for example.

In general, the reduced spectral CT dataset 120' may be transmitted via any form of data communication in the operation S140. For instance, the reduced spectral CT dataset 120' may be transmitted via wired, or optical, or wireless communication. By way of some examples, when wired or optical communication is used, the communication may take place via signals transmitted on an electrical or optical cable, and when wireless communication is used, the communication may take place via RF or optical signals.

An example of the operation S 140 is illustrated in Fig. 3. In this example, the reduced spectral CT dataset 120' is transmitted to a cloud-based second processing system 150 and/or to a cloud-based computer-readable storage medium 160.

As mentioned above, in contrast to transmitting the entire spectral CT dataset 120, the transmission of the reduced spectral CT dataset 120' in the operation S 140 alleviates bandwidth limitations and/or delays incurred in transmitting the data.

Various further examples of the present disclosure are described below. In one example, the method illustrated in Fig. 1 includes transmitting S140 the reduced spectral CT dataset 120' to the second processing system 150; and the method also includes: performing, using the second processing system 150, a second haemodynamic simulation using at least a portion of the reduced spectral CT dataset 120' to provide a second haemodynamic simulation result.

In this example, the second haemodynamic simulation result may include a value of a haemodynamic parameter and/or a clinical diagnosis of the anatomical region 130.

In some examples, the second haemodynamic simulation is the same, or a similar, haemodynamic simulation to the first haemodynamic simulation. For example, the second haemodynamic simulation may be the same simulation as the first simulation, the second simulation simply being more accurate than the first haemodynamic simulation. Using the second processing system to obtain a second haemodynamic simulation result that is more accurate than the first haemodynamic simulation result alleviates the processing burden on the first processing system.

In other examples, the second haemodynamic simulation is a different haemodynamic simulation to the first haemodynamic simulation. For example, the first haemodynamic simulation may include solving a lumped parameter model using a machine learning-based approach to provide a first haemodynamic simulation result, e.g. a first FFR value for a vessel. The second haemodynamic simulation may include using CFD techniques to solve haemodynamic model to provide a second haemodynamic simulation result, e.g. a second FFR value for a vessel. The second haemodynamic simulation result may be more accurate than the first haemodynamic simulation result. Thus, the first processing system is used to provide an estimated FFR value, and this estimated FFR value is used to obtain a reduced spectral CT dataset, which after being transmitted to the second processing system is used to obtain a more accurate FFR value. This provides an accurate FFR value whilst also alleviating the processing burden on the first processing system.

The second processing system may support different types of spectral CT data processing to those supported by the first processing system. By transmitting the reduced spectral CT dataset to the second processing system, the reduced spectral CT dataset may be used to obtain the values of different haemodynamic parameters, and different clinical diagnoses, than those that are obtained using the first processing system.

In some examples, the second processing system has a relatively greater processing power than the first processing system. For instance, the first processing system may be local to the acquisition of the spectral CT data, and the second processing system may be a remote, e.g. a cloud-based, processing system that has relatively greater processing power. By transferring the reduced spectral CT dataset to the second processing system and performing the second haemodynamic simulation there, the processing burden on the first processing system may be alleviated, or the second haemodynamic simulation result may be provided more quickly and/or in a more accurate manner than by using the first processing system.

In one example, after having performed the second haemodynamic simulation using the second processing system 150, the method includes transmitting the second haemodynamic simulation result from the second processing system 150 to the first processing system 110, or to a third processing system. This makes the second haemodynamic simulation result available at these processing systems respectively.

The second haemodynamic simulation result also be outputted by the second processing system 150, or by the third processing system. The second haemodynamic simulation result may be outputted in various ways, including to a display device such as to the monitor 220 illustrated in Fig. 2, or to a virtual/ augmented reality display device, or to a printer, or to a computer-readable storage medium, or to the Internet, or to the Cloud, and so forth. The first haemodynamic simulation result may also be outputted in a similar manner.

In one example, the first processing system is used to identify data processing operations that are to be performed using the second processing system. The second processing system then uses the data processing operation(s) to provide the second haemodynamic simulation result. In this example, the method illustrated in Fig. 1 includes:
- identifying, using the first processing system 110, one or more data processing operations to be performed by the second processing system 150;
- transmitting an indication of the one or more data processing operations to the second processing system 150; and
- wherein the performing a second haemodynamic simulation comprises applying the one or more data processing operations to the at least a portion of the reduced spectral CT dataset 120' to provide the second haemodynamic simulation result.

In this example, the use of the first processing system to identify the data processing operation(s) facilitates automated processing of the at least a portion of the reduced spectral CT dataset 120' by the second processing system.

The data processing operation(s) may be identified in various ways. In one example, the data processing operation(s) are identified by analysing the spectral CT data to provide a clinical diagnosis of the anatomical region. For instance, the spectral CT data may be analysed to detect the presence of vascular plaque in the anatomical region. If diffuse plaque patterns are present, then a spectral analysis of the plaque properties will likely be performed by the second processing system as part of a more detailed analysis. Thus, in this example, the data processing operation "perform spectral analysis of plaque properties" may be identified, and transmitted to the second processing system.

In another example, instead of, or in addition to identifying the data processing operations by analysing the spectral CT data to provide a clinical diagnosis of the anatomical region, the data processing operations may be identified based on the first haemodynamic simulation result. For instance, if the first haemodynamic simulation result is an FFR value that is evaluated by the first processing system using a lumped parameter model, then if the FFR value is below a specified value, the data processing operation "perform second haemodynamic simulation using a more accurate model (e.g. a haemodynamic model that is to be solved using CFD techniques)" may be identified, and transmitted to the second processing system. Similarly, if the first haemodynamic simulation result is an indication that calcified plaque is present in a vessel, then the data processing operation "perform second haemodynamic simulation using a more accurate model within the calcified vessel" may be identified.

In a related example, the identified data processing operations are further used to obtain the reduced spectral CT dataset 120' from the spectral CT data 120. Thus, in this example the reduced spectral CT dataset 120' is obtained from the spectral CT data 120 based on the identified data processing operations as well as the relevance of one or more subsets 120_{1..i} of the spectral CT data to the first haemodynamic simulation result 140.

This example has the advantage of ensuring that the reduced spectral CT dataset 120' contains sufficient information to perform the identified data processing operations. In this example, the identified data processing operations may place a minimum requirement/ requirements on the reduced spectral CT dataset 120'. For instance, the identified data processing operations may place a minimum requirement on which anatomical regions are represented by the reduced spectral CT dataset 120', or it may place a minimum requirement on the image quality (i.e. maximum amount of compression) of anatomical region(s) that are represented by the reduced spectral CT dataset 120'. In some cases, this may involve augmenting a reduced spectral CT dataset 120' that is obtained based on the relevance of one or more subsets 120_{1..i} of the spectral CT data to the first haemodynamic simulation result 140, with additional spectral CT data representing one or more further anatomical regions. Alternatively, or additionally, it may involve reducing the amount of compression that is applied to an anatomical region in the reduced spectral CT dataset 120'.

The data processing operation(s) in these examples may be identified using various technique. These include using a look-up table, and using a trained machine learning model. The look-up table provides for each of multiple conditions (e.g. results of the first haemodynamic simulation, clinical diagnoses, identified data processing operations, and so forth), the corresponding data processing operation(s). The trained machine learning model is trained to generate the data processing operation(s) in response to the corresponding inputted conditions.

In another example, patient data corresponding to the anatomical region is used to perform the first and/or the second haemodynamic simulation. In this example, the method illustrated in Fig. 1 includes: receiving, by the first processing system 110 and/or the second processing system 150, patient data 170 corresponding to the anatomical region 130.

In this example, the operation of performing the first haemodynamic simulation and/or performing the second haemodynamic simulation, using the first processing system 110 and/or the second processing system 150, respectively, is performed using the received patient data 170.

In this example, the patient data may include data such as blood pressure data, or heart rate data, for example. Such patient data may be obtained from the spectral CT data, e.g. by analysing reconstructed spectral CT images, or it may be obtained from interventional devices that are coupled to patient during acquisition of the spectral CT data. For example, the patient data may be obtained from an injector that is used to inject contrast agent into a s subject. Such data may be used as boundary conditions in the first/ second haemodynamic simulations. Using the patient data 170 in this manner helps to improve the accuracy of the first/ second haemodynamic simulation.

In one example, the method illustrated in Fig. 1 includes outputting the first haemodynamic simulation result 140. The first haemodynamic simulation result may be outputted in various ways, such as to a display device (e.g. the monitor 220 illustrated in Fig. 2), or to a virtual/ augmented reality display device, or to a printer, or to a computer-readable storage medium, or to the Internet, or to the Cloud, and so forth.

In one example, an image corresponding to the spectral CT data is outputted. The image corresponding to the spectral CT data may be outputted in a similar manner to the first haemodynamic simulation result 140.

In one example, an image corresponding to the reduced spectral CT dataset 120' is outputted. The image corresponding to the reduced spectral CT dataset 120' may be outputted in a similar manner to the first haemodynamic simulation result 140.

In one example, one or more further techniques may be applied to the spectral CT data 120, or to the reduced spectral CT dataset 120', in order to further reduce its size. For instance, known techniques may be used to remove redundant data that does not represent the anatomy, or to select data corresponding to a specific portion of the cardiac/ breathing cycle.

In one example, a computer program product, is provided. The computer program product comprises instructions which when executed by one or more processors, cause the one or more processors to carry out a method of performing a haemodynamic simulation. The method includes:
- receiving S110, by a first processing system 110, spectral CT data 120 representing an anatomical region 130;
- performing S120, using the first processing system 110, a first haemodynamic simulation using at least a portion of the spectral CT data 120 to provide a first haemodynamic simulation result 140;
- obtaining S130 a reduced spectral CT dataset 120' from the spectral CT data 120 based on a relevance of one or more subsets 120_{1..i} of the spectral CT data to the first haemodynamic simulation result 140; and
- transmitting S140 the reduced spectral CT dataset 120' to a second processing system 150 and/or to a computer-readable storage medium 160.

In one example, a system for performing a haemodynamic simulation, is provided. The system includes a first processing system 110 configured to:
- receive S110 spectral CT data 120 representing an anatomical region 130;
- perform S120 a first haemodynamic simulation using at least a portion of the spectral CT data 120 to provide a first haemodynamic simulation result 140;
- obtain S130 a reduced spectral CT dataset 120' from the spectral CT data 120 based on a relevance of one or more subsets 120_{1..i} of the spectral CT data to the first haemodynamic simulation result 140; and
- transmit S140 the reduced spectral CT dataset 120' to a second processing system 150 and/or to a computer-readable storage medium 160.

An example of the system is illustrated in Fig. 2. It is noted that in addition to the first processing system 110, the system may also include one or more of: a spectral X-ray imaging system for providing the spectral CT data, such as the spectral CT imaging system 210 illustrated in Fig. 2; a display, such as the monitor 220 illustrated in Fig. 2, for displaying the first haemodynamic simulation result 140, the second haemodynamic simulation result, an image representing the spectral CT data 120, an image representing the reduced spectral CT dataset 120', other outputs generated by the first/ second processing system, and so forth; a communication device (not illustrated in Fig. 2) for transmitting the reduced spectral CT dataset 120', and other transmitted data; an injector 230 for injecting a contrast agent; a patient bed; and a user input device (not illustrated in Fig. 2) configured to receive user input in relation to the operations performed by the first/ second processing system, such as a keyboard, a mouse, a touchscreen, and so forth.

In one example, a processing arrangement 200 for performing a haemodynamic simulation, is provided. The processing arrangement includes:
- a first processing system 110; and
- a second processing system 150 and/or a computer-readable storage medium 160;
- wherein the first processing system 110 is configured to:
   receive S110 spectral CT data 120 representing an anatomical region 130;
   perform S120 a first haemodynamic simulation using at least a portion of the spectral CT data 120 to provide a first haemodynamic simulation result 140;
   obtain S130 a reduced spectral CT dataset 120' from the spectral CT data 120 based on a relevance of one or more subsets 120_{1..i} of the spectral CT data 120 to the first haemodynamic simulation result 140; and
   transmit S140 the reduced spectral CT dataset 120' to the second processing system 150 and/or to the computer-readable storage medium 160.

An example of the processing arrangement 200 is illustrated in Fig. 2. It is noted that in addition to the first processing system 110 and the second processing system 150 and/or a computer-readable storage medium 160, the processing arrangement 200 may also include one or more of: a spectral X-ray imaging system for providing the spectral CT data, such as the spectral CT imaging system 210 illustrated in Fig. 2; one or more displays, such as the monitor 220 illustrated in Fig. 2, for displaying the first haemodynamic simulation result 140, the second haemodynamic simulation result, an image representing the spectral CT data 120, an image representing the reduced spectral CT dataset 120', other outputs generated by the first/ second processing system, and so forth; one or more communication devices (not illustrated in Fig. 2) for transmitting the reduced spectral CT dataset 120', and other transmitted data; an injector 230 for injecting a contrast agent; a patient bed; and one or more user input device(s) (not illustrated in Fig. 2) configured to receive user input in relation to the operations performed by the first/ second processing system, such as a keyboard, a mouse, a touchscreen, and so forth.

The above examples are to be understood as illustrative of the present disclosure, and not restrictive. Further examples are also contemplated. For instance, the examples described in relation to a computer-implemented method, may also be provided by the computer program product, or by the computer-readable storage medium, or by the system, or by the processing arrangement 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of performing a haemodynamic simulation, the method comprising:
receiving (S110), by a first processing system (110), spectral CT data (120) representing an anatomical region (130);
performing (S120), using the first processing system (110), a first haemodynamic simulation using at least a portion of the spectral CT data (120) to provide a first haemodynamic simulation result (140);
obtaining (S130) a reduced spectral CT dataset (120') from the spectral CT data (120) based on a relevance of one or more subsets (120_{1..i}) of the spectral CT data to the first haemodynamic simulation result (140); and
transmitting (S140) the reduced spectral CT dataset (120') to a second processing system (150) and/or to a computer-readable storage medium (160).

2. The computer-implemented method according to any previous claim, wherein the method comprises transmitting (S140) the reduced spectral CT dataset (120') to the second processing system (150); and
wherein the method further comprises:
performing, using the second processing system (150), a second haemodynamic simulation using at least a portion of the reduced spectral CT dataset (120') to provide a second haemodynamic simulation result.

3. The computer-implemented method according to claim 2, wherein the method further comprises transmitting the second haemodynamic simulation result from the second processing system (150) to the first processing system (110), or to a third processing system.

4. The computer-implemented method according to any one of claims 1-3, wherein the obtaining (S130) a reduced spectral CT dataset (120') from the spectral CT data (120), comprises determining a relevance of the one or more subsets (120_{1..i}) of the spectral CT data (120) to the first haemodynamic simulation result (140); and
selecting, based on the relevance of the one or more subsets (120_{1..i}), at least one of the one or more subsets to provide the reduced spectral CT dataset (120').

5. The computer-implemented method according to any one of claims 1-3, wherein the obtaining (S130) a reduced spectral CT dataset (120') from the spectral CT data (120) comprises determining a relevance of the one or more subsets (120_{1..i}) of the spectral CT data (120) to the first haemodynamic simulation result (140); and
compressing at least a portion of the spectral CT data (120) based on the relevance of the one or more subsets (120_{1..i}) to provide the reduced spectral CT dataset (120').

6. The computer-implemented method according to any previous claim, wherein the performing (S120) a first haemodynamic simulation comprises:
applying one or more segmentation operations to the at least a portion of the spectral CT data (120) to identify one or more anatomical structures (120_{1..i}) in the anatomical region (130); and
calculating a value of a haemodynamic parameter for at least one of the one or more anatomical structures (120_{1..i}) to provide the first haemodynamic simulation result (140).

7. The computer-implemented method according to any one of claims 1-5, wherein the performing (S120) a first haemodynamic simulation comprises:
inputting the at least a portion of the spectral CT data (120) into a machine learning model; and
generating the first haemodynamic simulation result (140) in response to the inputting, using the machine learning model; and
wherein the machine learning model is trained to generate the first haemodynamic simulation result (140) based on the inputted spectral CT data (120).

8. The computer-implemented method according to any previous claim, wherein the method further comprises generating a heatmap indicating a relative importance of one or more anatomical structures in the anatomical region (130) to the first haemodynamic simulation result (140) and/or a relative importance of one or more energy intervals of the spectral CT data (120) to the first haemodynamic simulation result (140); and
wherein the obtaining (S130) a reduced spectral CT dataset (120'), comprises using the heatmap to determine the relevance of the one or more subsets (120_{1..i}) of the spectral CT data (120) to the first haemodynamic simulation result (140).

9. The computer-implemented method according to claim 8 when dependent on claim 7, wherein the machine learning model is further trained to generate the heatmap; and
wherein the method comprises:
generating the heatmap in response to the inputting, using the machine learning model.

10. The computer-implemented method according to claim 2, wherein the method further comprises:
identifying, using the first processing system (110), one or more data processing operations to be performed by the second processing system (150);
transmitting an indication of the one or more data processing operations to the second processing system (150); and
wherein the performing a second haemodynamic simulation comprises applying the one or more data processing operations to the at least a portion of the reduced spectral CT dataset (120') to provide the second haemodynamic simulation result.

11. The computer-implemented method according to any previous claim, wherein the first haemodynamic simulation result (140), or the second haemodynamic simulation result, comprises a value of a haemodynamic parameter and/or a clinical diagnosis of the anatomical region (130).

12. The computer-implemented method according to any previous claim, wherein the one or more subsets (120_{1..i}) of the spectral CT data (120) correspond to one or more anatomical structures in the anatomical region (130) and/or wherein the one or more subsets (120_{1..i}) of the spectral CT data (120) correspond to one or more energy intervals of the spectral CT data.

13. The computer-implemented method according to any previous claim, wherein the method further comprises receiving, by the first processing system (110) and/or the second processing system (150), patient data (170) corresponding to the anatomical region (130); and
wherein the performing, using the first processing system (110) and/or the second processing system (150), respectively, is performed using the received patient data (170).

14. A computer program product comprising instructions which when executed by one or more processors, cause the one or more processors to carry out the method according to any one of claims 1-13.

15. A processing arrangement (200) for performing a haemodynamic simulation, the processing arrangement comprising:
a first processing system (110); and
a second processing system (150) and/or a computer-readable storage medium (160);
wherein the first processing system (110) is configured to:
receive (S110) spectral CT data (120) representing an anatomical region (130);
perform (S120) a first haemodynamic simulation using at least a portion of the spectral CT data (120) to provide a first haemodynamic simulation result (140);
obtain (S130) a reduced spectral CT dataset (120') from the spectral CT data (120) based on a relevance of one or more subsets (120_{1..i}) of the spectral CT data (120) to the first haemodynamic simulation result (140); and
transmit (S140) the reduced spectral CT dataset (120') to the second processing system (150) and/or to the computer-readable storage medium (160).
